# EUROPEAN PATENT SPECIFICATION

(11) **EP 0 664 834 B1**
(45) Date of publication and mention of the grant of the patent: **21.04.1999**
(21) Application number: 93923003.3
(22) Date of filing: 15.10.1993
(51) Int. Cl.: C12N 15/13, C12P 21/08, A61K 39/395, A61K 47/48, C12N 5/10

(54) **RECOMBINANT HUMANIZED ANTI-CYTOMEGALOVIRUS ANTIBODIES**
REKOMBINANTE HUMANISIERTE ANTI-CYTOMEGALOVIRUS ANTIKÖRPER
ANTICORPS RECOMBINES ET HUMANISES DIRIGES CONTRE LE CYTOMEGALOVIRUS

(30) Priority: 15.10.1992 GB 9221654
(43) Date of publication of application: 02.08.1995
(73) Proprietor: SCOTGEN LIMITED, Bridge of Don, Aberdeen AB22 8GU (GB)
(72) Inventor: HARRIS, William Joseph, Carnoustie, Angus DD7 7AB (GB); CARR, Francis Joseph, Balmedie, Aberdeenshire AB4 0XU (GB); HAMILTON, Anita, Anne, Aberdeen AB2 6BQ (GB)
(74) Representative: Armitage, Ian Michael
(86) International application number: GB9302134
(87) International publication number: WO9409136

(56) References cited:
- EP-A- 0 248 909
- EP-A- 0 277 071
- WO-A-91/09967
- WO-A-92/11018
- WO-A-92/11383
- NATURE vol. 332 , 24 March 1988 , LONDON, GB pages 323 - 327 L. RIECHMANN ET AL. 'Reshaping human antibodies for therapy.'

## Description

The present invention relates to recombinant immunoglobulins specifically neutralising for human cytomegalovirus (HCMV) . These immunoglobulins were produced by combination of the complementarity determining regions (CDRs) from a non-human antibody with variable region frameworks and constant regions from a human antibody. The invention also discloses methods for the production of recombinant immunoglobulins against HCMV. The invention also discloses a region on the HCMV glycoprotein gH which is a target for neutralising antibodies and is highly conserved between different isolates of HCMV from patients.

Human cytomegalovirus is a DNA virus, a member of the Herpesvirus group that causes a ubiquitous mild or inapparent disease in normal individuals. The virus is not eliminated from the body after a primary infection, but persists in the form of a low grade chronic infection or remains in a latent state, so that reactivation may occur at a later stage. However HCMV may cause severe disease in immunocompromised subjects or those with immature immune systems, such as an often fatal pneumonitis in transplant recipients, sight-threatening retinitis in AIDS patients or mental retardation in neonates. HCMV is also able to cross the placenta and infect the developing fetus. A syndrome similar to that caused by congenital Rubella may result, especially if the infection takes place in the first trimester.

Although laboratory strains of the virus have been developed, these are not considered to have potential as vaccines due to the possibility of latency and reactivation of the virus and also the potential oncogenicity of Herpesviruses. Hyperimmune gamma globulin has been used successfully for prophylaxis and treatment of HCMV infections in transplant patients (Snydman, D.R. et al, (1987), N. Engl. J. Med., 317, 1049-1054). However, supplies of active hyperimmune globulin are limited, costs of such preparations are very high and the possibility of human pathogens remaining in the preparation exists. It is expected that administration of a pure antibody against the virus should be even more beneficial as the composition of the preparation will be defined, problems of variability between different gamma globulin formulations will be avoided, and the content of specific antiviral antibody will be greater. Murine monoclonal antibodies provide a source of specific antibodies which can be purified in large quantities and guaranteed free of contamination by human pathogens such as the hepatitis or HIV viruses. However although murine monoclonal antibodies have been used in some human therapies, these are not ideal due to the development of an immune response to the "foreign" mouse proteins, termed the human anti-mouse antibody or HAMA response (Schroff, R., et al, (1985), Cancer Res., 45, 879-885), which in some cases causes serum sickness and leads to the murine antibodies being rapidly cleared from the circulation. These immune responses in humans have been shown to be against both the variable and the constant regions of the murine immunoglobulins. Recombinant DNA technology has provided the ability to alter antibodies in order to substitute specific immunoglobulin (Ig) regions from one species with regions from another. Patent Cooperation Treaty Patent Application No. PCT/GB85/00392 (Neuberger et al and Celltech Ltd.) describes a process whereby the complementary heavy and light chain variable domains of an Ig molecule from one species may be combined with the complementary heavy and light chain Ig constant domains from another species. This process may be used to substitute the murine constant region domains of a monoclonal antibody with human IgG constant region domains to create a "chimeric" antibody which could be used for human therapy. Such a chimeric antibody would have the advantage of having the human Fc region for efficient stimulation of antibody mediated effector functions, such as complement fixation, but would still have the potential to elicit an immune response in humans against the murine ("foreign") variable regions.

British Patent Application Number GB2188638A (Winter) describes a process whereby antibodies are altered by substitution of their complementarity determining regions (CDRs) with those from another species. This process may be used to substitute the CDRs from the murine variable region domains of a monoclonal antibody with desirable binding properties (for instance to a human pathogen) into human heavy and light chain Ig variable region domains. These altered Ig variable regions could then be combined with human Ig constant regions to create antibodies which are totally human in composition except for the substituted murine CDRs. Such "reshaped" or "humanised" antibodies should elicit a considerably reduced immune response in humans compared to chimeric antibodies as they contain considerably less murine components and their half life in the circulation should approach that of natural human antibodies. However, as stated in British Patent Application Number GB2188638A, merely replacing the CDRs with complementary CDRs from another antibody which is specific for an antigen such as a viral or bacterial protein, does not always result in an altered antibody which retains the desired binding capacity. In practice some amino acids in the framework of the antibody variable region interact with the amino acid residues that make up the CDRs so that amino acid substitutions into the human Ig framework regions are likely to be required to restore antigen binding. British Patent Application Number 9019812.8 describes a method for finding a minimal number of substitutions of foreign residues consistent with efficient binding to antigen.

Monoclonal antibodies have been developed against various HCMV glycoproteins which are found both in the viral envelope and in the membranes of infected cells and are important targets for the human immune system. Of particular importance are antibodies against a 86 kilodalton viral glycoprotein termed gH (Cranage, M.P. et al, (1988), Journal of Virology, 62, 1416-1422). Glycoprotein gH is thought to be involved in viral penetration and cell-to-cell spread and a cell surface receptor for this glycoprotein has been identified (Keay S. et al, (1989), Proc. Natl. Acad. Sci. USA, 86, 10100-10103). Thus, glycoprotein gH has been recognised as a target for neutralising antibodies which block virus penetration and cell-to-cell spread.

There is a need for the development of new therapies for the prevention and treatment of HCMV infections, particularly in immunocompromised patients. A monoclonal antibody specific for a neutralising epitope of HCMV would provide such a new therapy. The present invention provides a monoclonal antibody specific for HCMV gH which has been converted to a "human" antibody by the use of "reshaping" technology to confer all the advantages of low immunogenicity, long half life and human effector functions of human antibodies with the specificity and ease of production and purification of murine monoclonal antibodies, to be used in the treatment of HCMV infections.

Accordingly, in one aspect, the present invention provides a monoclonal antibody, or antigen-binding fragment thereof, as set out in claim 6. These antibodies and fragments can be made according to the method set out in claim 1.

### BRIEF DESCRIPTION OF THE FIGURES

Figure 1 shows the DNA sequence and corresponding amino acid sequence of the HCMV16 heavy chain variable region (VH). The CDR sequences are boxed. The first eight and the last eleven amino acids, as underlined, correspond to the sequences of the oligonucleotide primers used.
Figure 2 shows the DNA sequence and corresponding amino acid sequence of the HCMV16 light chain variable region (VK). The CDR sequences are boxed. The first eight and the last six amino acids, as underlined, correspond to the sequences of the oligonucleotide primers used.
Figure 3 shows the vector pSVgpt for expression of reshaped or chimeric heavy chains in mammalian cells.
Figure 4 shows the vector pSVhyg for expression of reshaped or chimeric light chains in mammalian cells.
Figure 5 shows the variable region amino acid sequences HCMV16VH, 16HuVH , the modified HuVHs, 16HuVH ATAF, 16HuVH ATAFSTAY, 16HuVH ATAFFAFSL, 16HuVH ATAFKNE and 16HuEUVH YTR, HCMV16VK and 16HuVK. Altered framework residues are shown in lower case.
Figure 6 shows the variable region nucleotide sequences of 16HuVH, 16HuVH ATAF, 16HuVH ATAFSTAY, 16HuVH ATAFAFSL, 16HuVH ATAFKNE, 16HuEUVH YTR and 16HuVK.

### DETAILED DESCRIPTION OF THE INVENTION

As used herein, the term "reshaped" antibody refers to a molecule wherein the complementarity determining regions (CDRs) are derived from an immunoglobulin from a non-human species, the remaining parts of the antibody molecule being derived mainly from human immunoglobulin.

The present invention relates to altered antibodies in which at least parts of the complementarity determining regions (CDRs) in the light and/or heavy chain variable domains of an acceptor human antibody have been replaced by analagous parts of CDRs of one or more donor monoclonal antibodies, whereby there is minimal alteration of the acceptor antibody heavy and/or light variable domain framework region in order to retain donor monclonal antibody binding specificity, whereby the donor monoclonal antibody has specificity for the human cytomegalovirus (HCMV) gH glycoprotein. The invention also relates to a region on gH, recognised by the altered antibodies, which is highly conserved between clinical strains of the virus. It will be recognised that it is possible to use the murine HCMV16 antibody or the altered antibodies of the invention to identify this region within gH, using procedures to subdivide or modify the gH sequence and to investigate the effect of those subdivisions or modifications on recognition by the antibody. Such procedures include defined amino acid substitutions and/or deletions and/or insertions into the gH sequence e.g. Qadri I. et al (1992) J. Gen. Immunol., 73, 2913-2921; the use of peptide synthesis or gene expression of subsequences of the gH gene in a suitable host to obtain sets of defined or random peptides which encompass the gH protein sequence e.g. Geysen, H.M. et al (1987) J. Immunol. Methods 102, 259-274, Scott, J.K. and Smith, G.P. (1990) Science 249, 386-390.

Alternatively or in conjunction with the above it is possible to determine the gH gene sequence or protein sequence for viruses which have lost the property of being recognised by the murine HCMV16 antibody or the altered antibodies of the invention. Such sequences should identify regions of gH which contribute to the epitope recognised by the antibodies e.g. Pellett, P.E. et al (1985) J. Virology 53, 243-253.

Preferably the altered antibodies will be produced by recombinant DNA technology. The present invention also relates to the DNA and protein sequence coding for such antibodies. These antibody genes comprise DNA coding for the human Ig constant region and variable framework region domains and one or more CDRs from a different source. In addition, amino acid substitutions in the human variable region frameworks are described which are critical for antigen binding affinity. The minimal number of substitutions are made so that a large-scale introduction of non-human framework residues is avoided. The invention also provides vectors for the production of the altered antibodies in mammalian cell hosts.

The altered antibodies of the invention may be produced by the following process:
(a) constructing, by conventional techniques, an expression vector containing an operon with a DNA sequence encoding an antibody heavy chain in which the CDRs and such minimal portions of -the variable domain framework region that are required to retain donor antibody binding specificity are derived from a non-human immunoglobulin, such as that produced by HCMV16, and the remaining parts of the antibody chain are derived from a human immunoglobulin, thereby producing the vector of the invention;
(b) constructing, by conventional techniques, an expression vector containing an operon with a DNA sequence encoding a complementary antibody light chain in which the CDRs and such minimal portions of the variable domain framework region that are required to retain donor antibody binding specificity are derived from a non-human immunoglobulin, such as that produced by HCMV16, and the remaining parts of the antibody chain are derived from a human immunoglobulin, thereby producing the vector of the invention;
(c) transferring the expression vectors to a host cell by conventional techniques to produce the transfected host cell of the invention;
(d) culturing the transfected cell by conventional techniques to produce the altered antibody of the invention.

The host cell may be cotransfected with the two vectors of the invention or these may be transfected sequentially; the first vector containing an operon encoding a light chain derived polypeptide and the second vector containing an operon encoding a heavy chain derived polypeptide. The two vectors contain different selectable markers, but otherwise, apart from the antibody heavy and light chain coding sequences, are preferably identical, to ensure, as far as possible, equal expression of the heavy and light chain polypeptides. Alternatively, a single vector may be used, the vector including the sequences encoding both the light and the heavy chain polypeptides. The coding sequences for the light and heavy chains may comprise cDNA or genomic DNA or both.

The host cell used to express the altered antibody of the invention may be either a bacterial cell such as Escherichia coli, or preferably a eukaryotic cell. In particular a mammalian cell of a well defined type for this purpose, such as a myeloma cell or a Chinese hamster ovary cell may be used.

The general methods by which the vectors of the invention may be constructed, transfection methods required to produce the host cell of the invention and culture methods required to produce the antibody of the invention from such host cells are all conventional techniques. Likewise, once produced, the altered antibodies of the invention may be purified according to standard procedures of the art, including cross-flow filtration, ammonium sulphate precipitation, affinity column chromatography, gel electrophoresis and the like.

The altered antibody of the present invention may comprise a complete antibody molecule, having full length heavy and light chains or any fragment thereof, such as the Fab or (Fab')₂ fragment, a light chain or heavy chain dimer, or any minimal fragment thereof such as an Fv or a SCA (single chain antibody) or any other molecule with the same specificity as the altered antibody of the invention. Alternatively the altered antibody of the invention may have attached to it an effector or reporter molecule, for instance a toxin such as ricin. Alternatively, one pair of heavy and light chains from the altered antibody may be associated with a heavy/light chain pair from another antibody to form a "bispecific" antibody. The constant region of the altered antibody may be derived from any human immunoglobulin isotype.

The present invention also relates to a process for preparing such altered antibodies; a pharmaceutical composition comprising a therapeutic, non-toxic amount of the altered antibodies and a pharmaceutically acceptable carrier or diluent. Examples of the altered antibodies of the invention are reshaped antibodies derived from the murine monoclonal antibody HCMV16 such as Reshaped HCMV16 ATAFSTAY or Reshaped HCMV16 EuYTR which are described in the examples. Such antibodies are useful in the treatment, either therapeutically or prophylactically, of HCMV infection. Therefore this invention also relates to a method of treating, therapeutically or prophylactically, an HCMV infection in a human in need thereof which comprises administering an effective dose of such altered antibodies to such a human.

The altered antibodies of this invention may be used in conjunction with other antibodies, particularly human or reshaped antibodies reactive with other markers or epitopes of the HCMV virus. Alternatively, the altered antibodies of this invention may be administered in conjunction with chemotherapeutic agents, such as the anti-viral drug gancyclovir, for the treatment or prevention of HCMV infections.

It should also be noted that the altered antibodies of this invention may be used for the design and synthesis of either peptide or non-peptide compounds (mimetics) which would be useful for the same therapy as antibody (Saragovi, H.U. et al (1991), Science, 253, 792-795).

The present invention applies to the provision of altered antibodies with the combination of properties for the treatment of HCMV infections in man. In particular, the invention provides "humanised" or "reshaped" antibodies which elicit a minimal immune response in humans, derived from a non-human antibody, which are specific for HCMV and have been shown to neutralise the virus.

The following examples are offered by way of illustration, not by limitation.

### EXAMPLES

In the following examples all necessary restriction and modification enzymes, plasmids and other reagents and materials were obtained from commercial sources unless otherwise indicated. In the following examples, unless otherwise indicated, all general recombinant DNA methodology was performed as described in "Molecular Cloning, A Laboratory Manual" (1982) Eds T.Maniatis et al, published by Cold Spring Harbor Laboratory Press, Cold Spring Harbor, New York.

In the following examples the following abbreviations may be employed:
- dCTP: deoxycytidine triphosphate
- dATP: deoxyadenosine triphosphate
- dGTP: deoxyguanosine triphoshate
- dTTP: deoxythymidine triphosphate
- DTT: dithiothreitol
- C: cytosine
- A: adenine
- G: guanine
- T: thymine
- PBS: phosphate buffered saline
- PBST: phosphate buffered saline containing 0.05% Tween 20 (pH7.5)

### EXAMPLE 1:- PRODUCTION OF ALTERED ANTIBODIES SPECIFIC FOR HCMV

The source of the donor CDRs used to prepare these altered antibodies was a murine monoclonal antibody, HCMV16, which is specific for the 86 kilodalton glycoprotein, gH, of HCMV. The HCMV16 hybridoma cell line was obtained from Bioscot Ltd., Edinburgh, U.K. and was produced by immunisation of mice with partially purified HCMV virus strain AD169 and subsequent production and screening of hybridoma cells.

Cytoplasmic RNA was prepared from the HCMV16 cell line by the method of Favoloro, J. et al, (1980), (Methods in Enzymology, 65, 718-749). cDNA was synthesised using Ig variable region primers as follows: for the Ig heavy chain variable region (VH), the primer VH1FOR (5'TGAGGAGACGGTGACCGTGGTCCCTTGGCCCCAG3'); for the light chain variable region (VK), the primer VK1FOR (5'GTTAGATCTCCAGCTTGGTCCC3'). cDNA synthesis reactions consisted of 10ug RNA, 20pmol VH1FOR or VK1FOR, 250 uM each of dATP, dCTP, dGTP and dTTP, 100mM TrisHCl pH8.3, 140mM KCl, 10mM DTT, 10mM MgCl₂ and 27 units of RNase inhibitor (Pharmacia, Milton Keynes, U.K.) in a total volume of 50 pl. Samples were heated to 70°C for 10 minutes (min) then slowly cooled to 42°C over a period of 30 min. 46 units of Moloney Murine Leukaemia virus (MMLV) reverse transcriptase (Anglian Biotec, Colchester, U.K) was added and incubation at 42°C continued for 1 hour.

VH and VK cDNAs were then amplified using the polymerase chain reaction (PCR) as described by Saiki, R.K. et al, (1988), Science, 239, 487-491. The primers used were: 
VH1FOR;

VK1FOR;

VH1BACK(5'AGGTSMARCTGCAGSAGTCWGG3')

VK1BACK(5'GACATTCAGCTGACCCAGTCTCCA3')
 where M = C or A, S = C or G, and W = A or T. These primers and their use in the PCR amplfication of mouse Ig DNA are described by Orlandi, R. et al, (1989), Proc. Natl. Acad. Sci. USA, 86, 3833-3837. For PCR amplification of VH, 5µl RNA/cDNA hybrid was mixed with 25pmol VH1FOR and VH1BACK primers. For PCR amplification of VK, 5µl RNA/cDNA hybrid was mixed with 25pmol VK1FOR and VK1BACK primers. To these mixtures was added 200uM each of dATP, dCTP, dGTP and dTTP, 67mM TrisHCl pH8.8, 17mM (NH₄)₂SO₄, 10mM MgCl₂, 0.02% (w/v) gelatin and 2 units of Taq polymerase (Perkin Elmer Ltd., Beaconsfield, U.K.). These were then subjected to 25 thermal cycles of PCR at 94°C, 1 min; 40°C, 1 min; 72°C, 2 min; ending with 5 min at 72°C. For cloning and sequencing, amplified DNA was purified by electrophoresis in a low melting point agarose gel and by Elutip-d column chromatography (Schleicher and Schuell, Dussel, Germany). Amplfied VH DNA was cut with PstI and BstEII and cloned into M13mp18 or M13mp19 cut with PstI and SmaI (Life Technologies, Paisley, U.K.). Amplified VK DNA was cut with PvuII and BglII and cloned into PvuII and BclI cut M13mp18 or M13mp19 (Life Technologies, Paisley, U.K.).

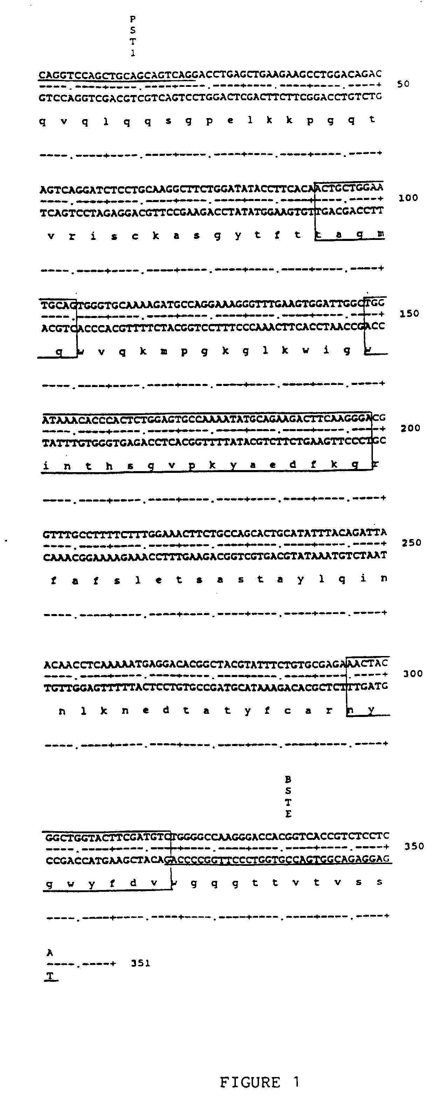

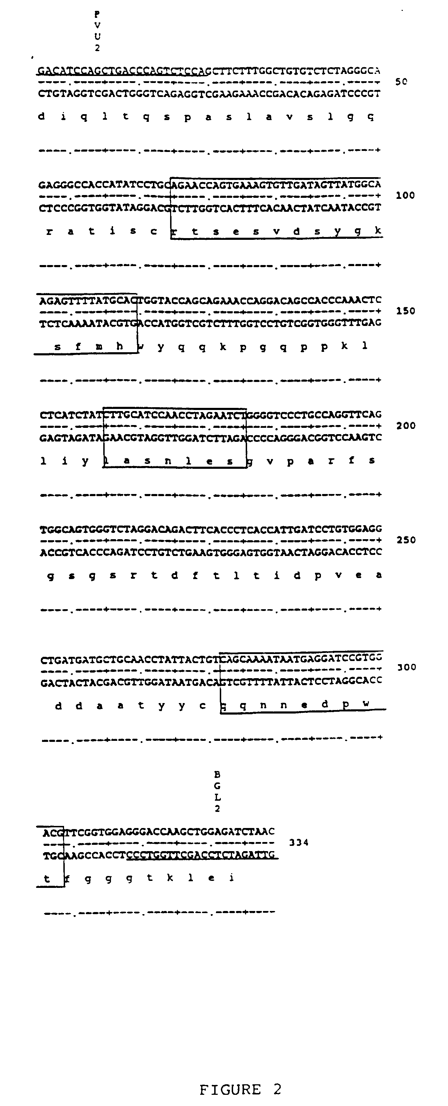

The resulting clones were sequenced by the dideoxy method (Sanger, F. et al, (1977), Proc. Natl. Acad. Sci. USA, 74, 5463-5467) using Sequenase (United States Biochemicals, Cleveland, Ohio, USA). The DNA and protein sequences of the HCMV16 VH and VK domains are shown in Figures 1 and 2. The location of the CDRs was determined with reference to Kabat, E.A. et al, (1987), "Sequences of Proteins of Immunological Interest", US Department of Health and Human Services, US Government Printing Office, and utilising computer assisted alignment with other VH and VK sequences.

The transfer of the murine CDRs to human frameworks was achieved by oligonucleotide site-directed mutagenesis, based on the method of Nakamaye, K. and Eckstein, F., (1986), Nucleic Acids Res, 14, 9679-9678. The primers used were: 
VHCDR1 5'CTGTCTCACCCACTGCATTCCAGCAGTGCTGAAGGTGTAGCCAGACAC
GGT3'
VHCDR2 5'CATTGTCACTCTTCCCTTGAAGTCTTCTGCATATTTTGGCACTCCAGA
GTGGGTGTTTATCCATCCAATCCACTC3'
VHCDR3 5'CCCTTGGCCCCAGACATCGAAGTACCAGCCGTAGTTTCTTGCACAAT
AAT3'
VKCDR1 5'CTGCTGGTACCAGTGCATAAAACTCTTGCCATAACTATCAACACTTT
CACTGGTTCTACAGGTGATGGTCACTCTG3'
VKCDR2 5'GCTTGGCACACCAGATTCTAGGTTGGATGCAAGGTAGATCAGCAG3'
VKCDR3 5'CCCTTGGCCGAACGTCCACGGATCCTCATTATTTTGCTGGCAGTA
GTAGGT3'
 The human framework regions chosen to receive the transplanted CDRs were NEWM and REI for the heavy and light chain respectively. The structures of these proteins have been crystallographically solved. The templates for mutagenesis were human framework region genes containing irrelevant CDRs and consisted of synthetic DNAs cloned into M13 phage (Riechmann, L. et al, (1988), Nature, 332, 323-327 ). The primer for transfer of VHCDR1 was extended to include one extra change at position 27. Tyrosine was substituted for serine which is an unusual amino acid at this position found in NEWM (see Riechmann et al,1988).

For site-directed mutagenesis the VH and VK oligonucleotides encoding the murine CDRs were phosphorylated with T4 kinase (Life Technologies Ltd., Paisley, U.K.). A two-fold molar excess of each of the three VH or VK primers were added to Sug of vH or VK single stranded template DNA in M13 (M13VHPCR1 or M13VKPCR2) and annealed by heating to 70°C for a few minutes and slowly cooling to 37°C. The annealed DNA was extended with 6 units of Klenow fragment of DNA polymerase 1 (Life Technologies Ltd., Paisley, U.K.) in a reaction mixture containing 6 units of DNA ligase (Life Technologies Ltd., Paisley, U.K.), 0.5mM of each of dATP, dGTP, dTTP and 2'-deoxycytidine 5'-0-(1-thiotriphosphate) (thiodCTP) (Pharmacia, Milton Keynes, U.K,), 60mM TrisHCl pH8.0, 6mM MgCl₂, 5mM DTT and 10mM ATP in a total volume of 50 µl. The mixture was incubated for 16 hours at 16°C then the DNA was precipitated with ethanol, redissolved and digested with 5 units of NciI (Life Technologies Ltd., Paisley, U.K.) for 90 min. This enzyme nicks the parental DNA strand, but leaves the newly synthesised strand containing the thiodCTP intact. The parental strand was removed by digestion with 100 units of exonuclease III (Life Technologies Ltd., Paisley, U.K.) for 30 min in 50 µl of 60mM TrisHCl pH8.0, 0.66mM MgCl₂ and lmM DTT then the DNA was repaired with 3 units of DNA polymerase I (Life Technologies Ltd., Paisley, U.K.) and 2 units of T4 DNA ligase in 50 µl of 60mM TrisHCl pH8.0, 6mM MgCl₂, 5mM DTT, 10mM ATP and 0.5mM each of dATP, dCTP, dGTP and dTTP, for 180 min at 16°C. The DNA was transformed into E.coli TG1 (Amersham International plc, Amersham, U.K.) made competent by the method of Simanis, described by Hanahan, D. (1985) in "DNA Cloning Volume 1" 109-135, Glover, D.M (ed), IRL Press. Single-stranded DNA was prepared from individual plaques and sequenced by the dideoxy method (see Maniatis et al, (1982), "Molecular Cloning, A Laboratory Manual"). If only single or double mutants were obtained these were subjected to further rounds of mutagenesis with the appropriate oligonucleotides until the required triple CDR mutants forming the reshaped VH and VK genes were obtained. A HindIII site in CDR3 of the VK was removed by site-directed mutagenesis using the primer 5'CACGGATCTTCATTATT3'. The amino acid sequence was not altered.

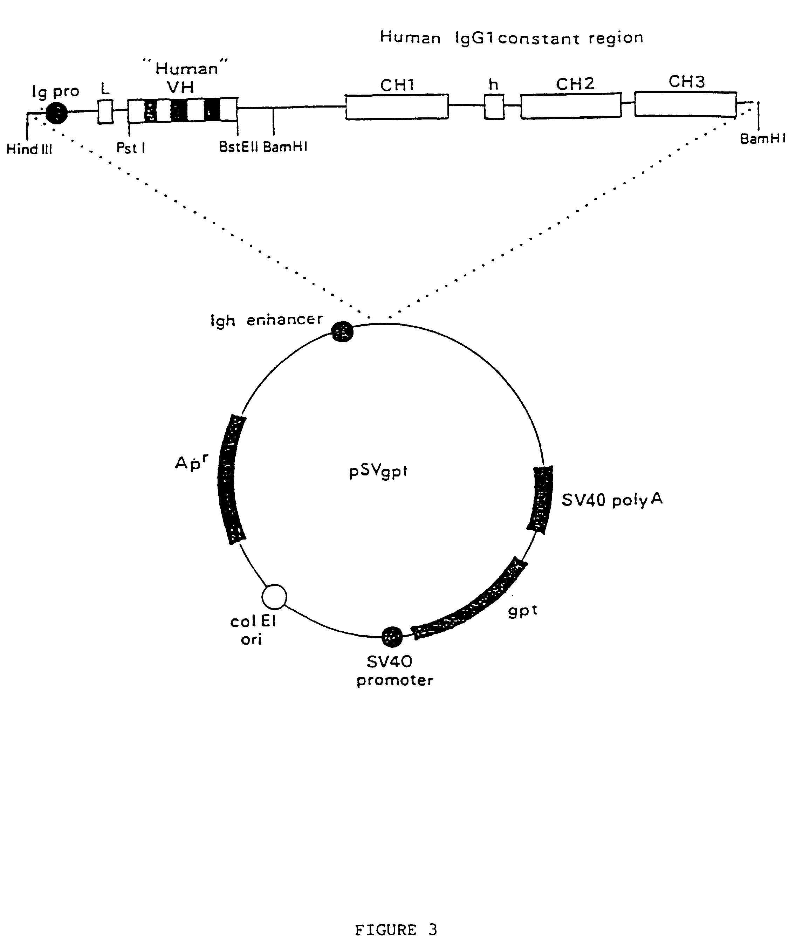

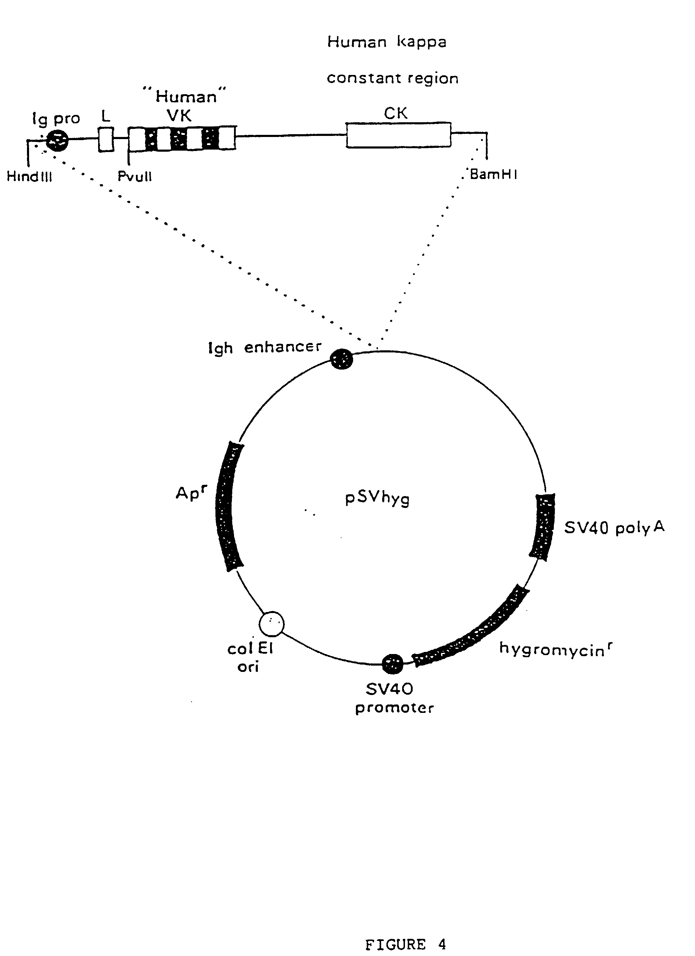

The expression vectors for the reshaped VH and VK genes, pSVgpt and pSVhyg are shown in Figures 3 and 4. The reshaped VH gene, together with the immunoglobulin heavy chain promoter, appropriate splice sites and signal peptide sequences was excised from M13 with HindIII and BamHI (Life Technologies Ltd., Paisley, U.K.) and cloned into the heavy chain expression vector, pSVgpt, which contains the murine heavy chain immunoglobulin enhancer, the gpt gene under control of the SV40 promoter/enhancer for selection in mammalian cells and sequences for replication and selection in E.coli. A human IgG1 constant region domain was added as a BamHI fragment and the correct orientation selected. The reshaped VK gene was cloned into the light chain expression vector, pSVhyg, in the same way except that the gpt gene is replaced by the gene for hygromycin resistance (hyg) and a human kappa constant region is included in the plasmid.

For transfection into mammalian cells, 10 ug of the heavy chain expression vector DNA and 20 ug of the light chain vector DNA were cut with PvuI (Life Technologies Ltd., Paisley, U.K.) to linearise, then precipitated with ethanol and redissolved in 25 µl of water. The recipient cell line was NSO, a non-immunoglobulin producing mouse myeloma, obtained from the European Collection of Animal Cell Cultures, Porton, U.K., ECACC No 85110505 or YB2/0, a non-immunoglobulin producing rat myeloma, obtained from the American Type Culture Collection, Rockville, Maryland, USA, ATCC No CRL 1662. Cells were grown in Dulbecco's Modified Eagle's Medium supplemented with 10% fetal calf serum and antibiotics (DMEM) (Life Technologies Ltd., Paisley, U.K.). Approximately 10⁷ NSO or YB2/O cells were harvested by centrifugation and resuspended in 0.5ml DMEM. The digested DNA was added and the cells transferred to a cuvette and placed on ice for 5 min. A single pulse of 170 volts, 960 ufarads was administered (Genepulser, BioRad, Richmond, California, USA). After a further 20 min on ice the cells were replaced in a flask in 20ml DMEM and allowed to recover for 48 hours. At this time the cells were distributed into a 24-well plate in selective medium (DMEM with 0.8 µg/ml mycophenolic acid and 250µg/ml xanthine). After 3 to 4 days the medium was changed for fresh selective medium so that dead cells were removed. Colonies of transfected cells were visible after 10 to 14 days.

The production of human antibody in the wells containing transfected clones was measured by ELISA. Capture antibody, goat anti-human IgG, gamma chain specific (Sera-Lab Ltd., Crawley Down, U.K.) was diluted to 5µg/ml in 50mM carbonate buffer pH9.6, and used to coat polystyrene ELISA plates (Dynatech Immulon 1), 200µl per well, overnight at 4°C. After washing 3 times with PBST, 50-100A, 25µl of the culture medium to be screened was added to the wells and incubated at 37°C for 60 min. The wells were washed again with PBST and the reporter antibody, peroxidase-conjugated goat anti-human IgG, gamma chain specific (Sera-Lab Ltd., Crawley Down, U.K.) or peroxidase-conjugated goat anti-human kappa chain (Sera-Lab Ltd., Crawley Down, U.K.) was added at 100ng per well and the plate incubated for a further 60 min. The plate was washed as before then the colour was developed. Substrate buffer was prepared by mixing 100mM citric acid and 100mM disodium hydrogen phosphate to pH5.0. 25mg of o-phenylenediamine was dissolved in 50ml and 5µl of 30% hydrogen peroxide added just before use. 200µl was dispensed per well and incubated at room temperature in the dark. The reaction was stopped by addition of 50µl per well of 12.5% sulphuric acid and the absorbances were read at 492nm.

Positive cell clones were expanded for antibody purification. For small scale purification 500ml of conditioned medium from static flask or spinner cultures was harvested by centrifugation. 0.1 volumes of 1.0M TrisHCl pH8.0 and 0.5 to 1.0ml of Protein A-agarose (Boehringer Mannheim, Lewes, U.K) were added. This was stirred overnight at room temperature then collected on a disposable column. This was washed with 10 column volumes of 0.1M TrisHCl pH8.0, 10 column volumes of 0.01M TrisHCl pH8.0 and eluted with 0.1M glycine buffer, pH3.0. 1.0ml fractions were collected into tubes containing 100µl of 1.0M TrisHCl, pH8.0. Antibody containing fractions were pooled and dialysed against PBS. The concentration of the antibody preparations was determined by measuring the absorbance at 280nm. Samples were checked by running on 10% SDS-polyacrylamide gels.

To improve the affinity of the reshaped antibody, 16HuVH/HuVK, the method described in British Patent Number 9019812.8 was adopted. The chimeric HCMV16 antibody, in which the murine constant region domains of the heavy and light chains had been replaced by the human constant regions used in the reshaped antibody, was constructed as described by Orlandi et al, (1989). The HindIII site in CDR3 of the VK was removed by site-directed mutagenesis as described above. Two hybrid chimeric/reshaped antibodies were constructed consisting of the chimeric heavy chain with the reshaped light chain and the reshaped heavy chain with the chimeric light chain. The first of these antibodies showed binding to and neutralisation of HCMV with equivalent efficiency to the chimeric antibody or original murine antibody. Therefore attention was directed towards the reshaped heavy chain in order to improve the affinity of the reshaped antibody.

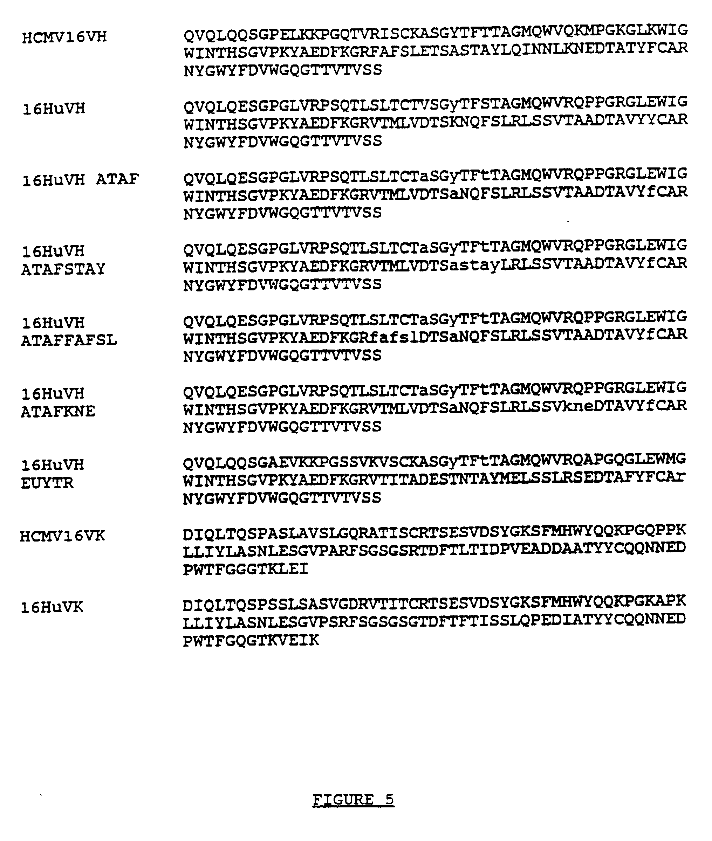

New versions of the reshaped heavy chain, 16HuVH, were designed. The amino acid sequences of these VH domains and the original murine HCMV16 VH, are shown in Figure 5. The first version, 16HuVH ATAF, had four changes; valine to alanine at position 24, serine to threonine at position 30, lysine to alanine at position 75 and tyrosine to phenylalanine at position 91 (numbering according to Kabat et al, loc. cit.). Three versions had additional substitutions to these; 16HuVH ATAFSTAY had serine, threonine, alanine and tyrosine substituted for asparagine, glutamine, phenylalanine and serine at positions 76 to 79; 16HuVH ATAFFAFSL had phenylalanine, alanine, phenylalanine, serine and leucine substituted for valine, threonine, methionine, leucine and valine at positions 67 to 71; 16HuVH ATAFKNE had lysine, asparagine and glutamic acid substituted for threonine, alanine and alanine at positions 83 to 85. Finally a version based on the human heavy chain EU was constructed. EU VH belongs to human heavy chain subgroup 1 (Kabat et al, loc. cit.) and has greater homology to murine HCMV16 VH than NEWM. Apart from the inclusion of the HCMV16 CDRs instead of the EU CDRs, three individual amino acid residues were altered; glycine to tyrosine at position 27, serine to threonine at position 30 and glycine to arginine at position 94; so that the residues immediately before CDRs 1 and 3 were as in the murine VH. Framework 4 was left as in the NEWM framework as the sequence given for EU is rather unusual.

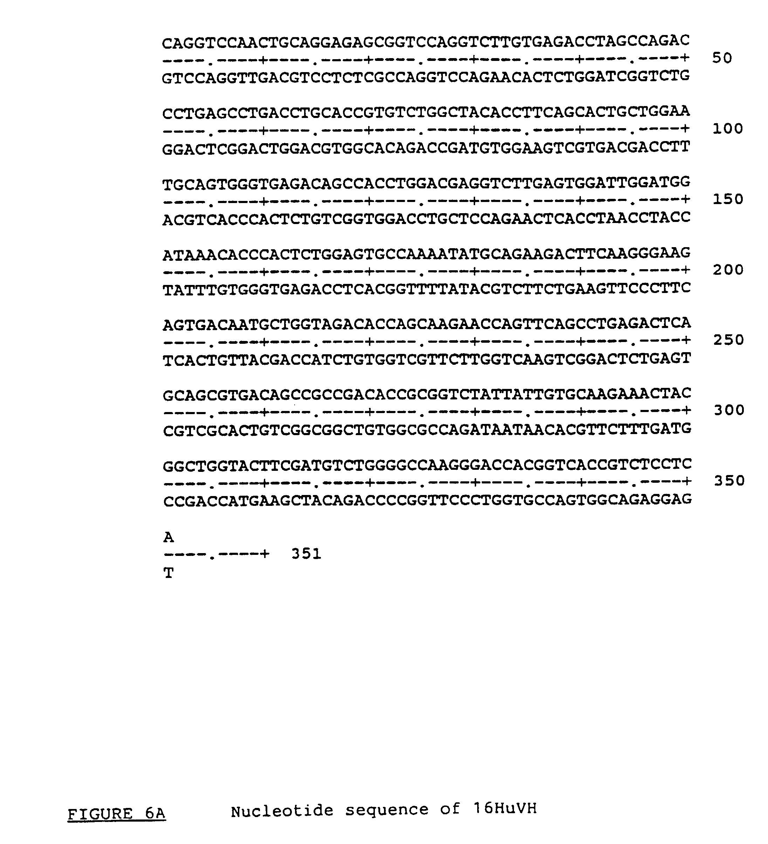

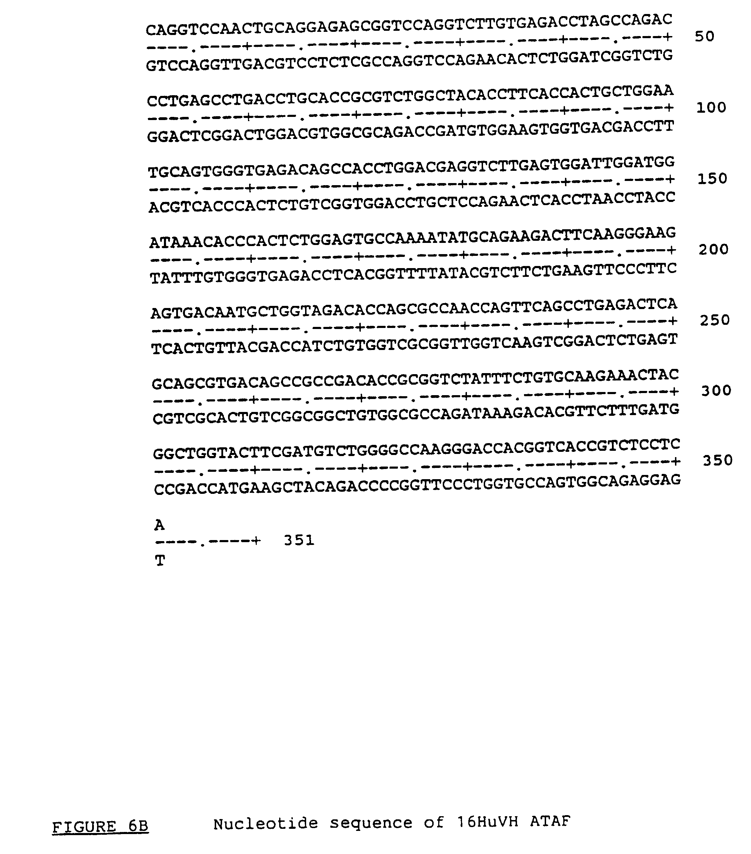

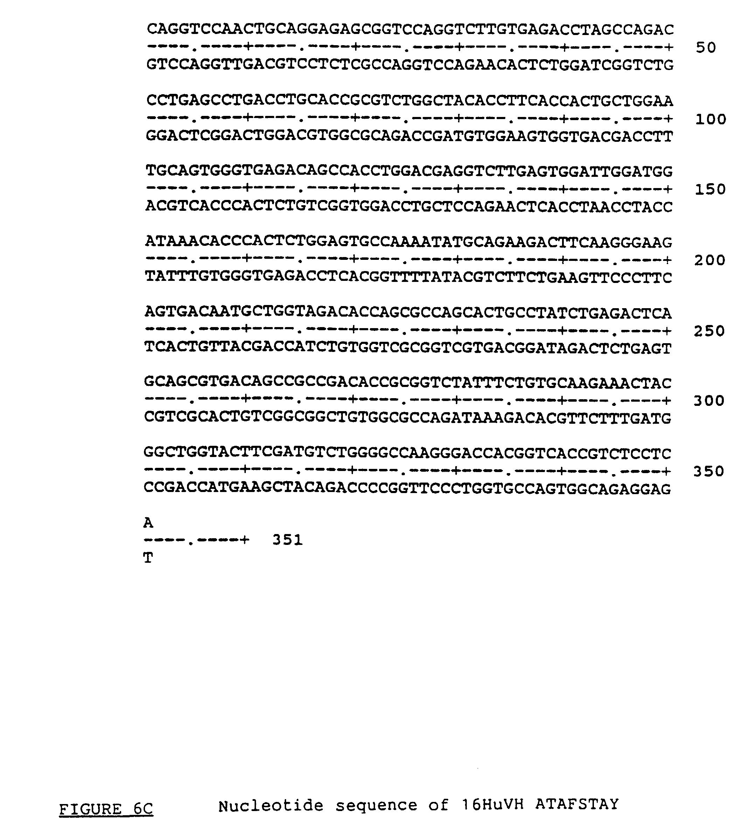

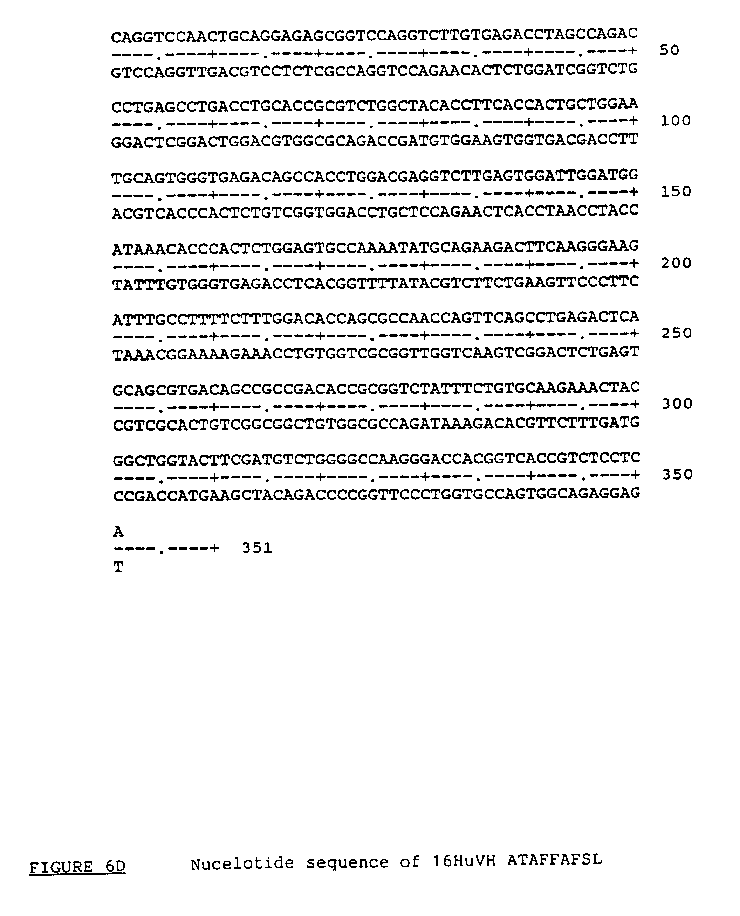

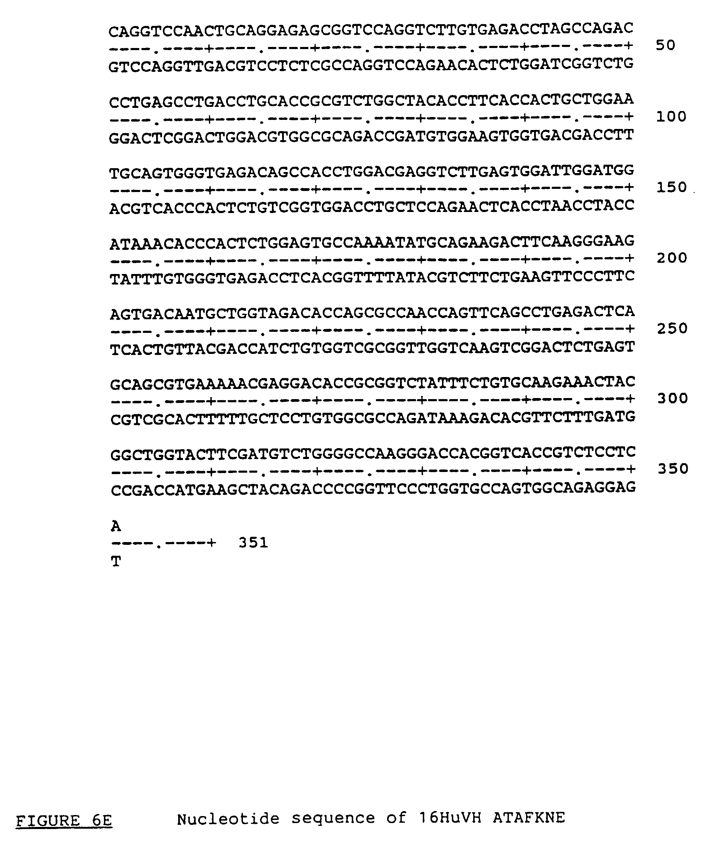

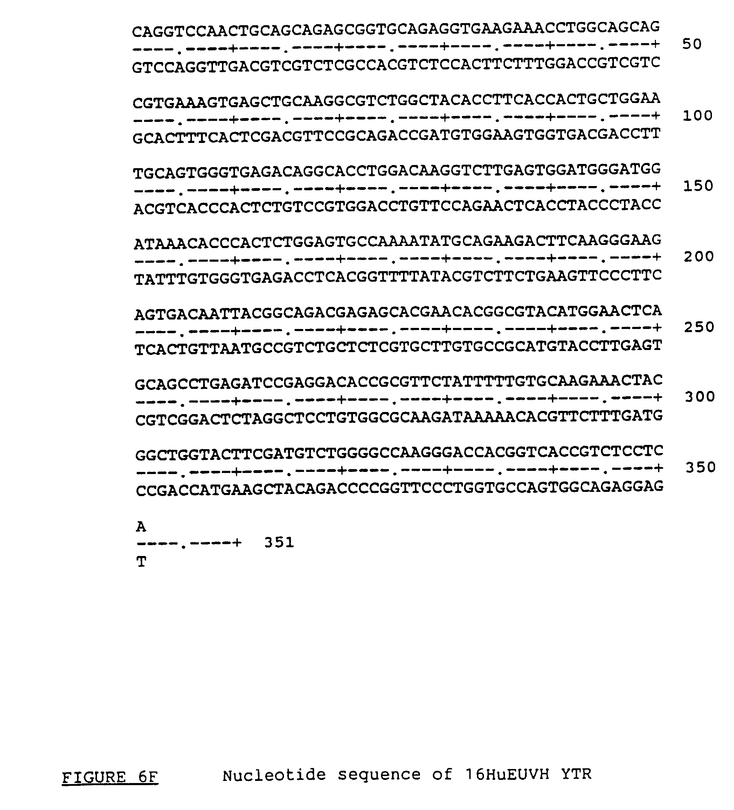

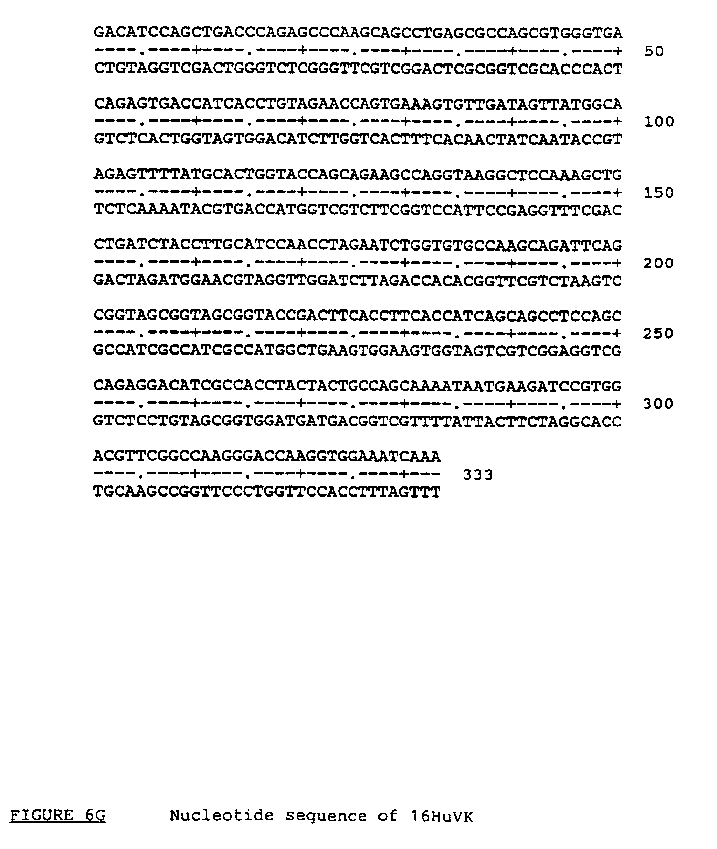

These new versions were constructed by mutagenesis of the original reshaped heavy chain in M13 phage. The method of Higuchi, R. et al, (1988), Nucleic Acids Res, 16, 7351-7367, which utilises overlapping PCR amplifications with mutagenic primers, was employed. The primers used to construct 16HuVH ATAF were; for the change at residue 24 from valine to alanine and at residue 30 from serine to threonine, 5'TCTGGCTACACCTTCACCACTGCTGGAATG3' and 5'GAAGGTGTAGCCAGACGCGGTGCAGGTCAG3'; for the change at the residue 75 from lysine to alanine 5'GACACCAGCGCCAACCAG TTCAGCC3' and 5'GGCTGAACTGGTTGGCGCTGGTGTC3'; and for the change at residue 91 from tyrosine to phenylalanine, 5'GACACCGCGGTCTATTTCTGTGCAAGAAACTAC3'and 5'GTAGTTTCTTGCACAGAAATAGACCGCGGTGTC3'. The 16HuvH ATAF DNA produced was used as template for the construction of 16HuVH ATAFSTAY, 16HuVH ATAFFAFSL and 16HuVH ATAFKNE. The primers used to construct 16HuVH ATAFSTAY were 5'GCTGGTAGACACCAGGCCAGCACTGCCTATCTGAGACTCAGCAGCG3' and 5'CGCTGCTGATCTCAGATAGGCAGTGCTGGCGCTGGTGTCTACCAGC3'. The primers used to construct 16 HuVH ATAFFAFSL were 5'GACACCAGCGCCAACCAGTTCAGCC3' and 5'GGCGCTGGTGTCCAAAGAAAAGGCAAATCTCCCTTGA3'. The primers used to construct 16HuVH ATAFKNE were 5'GACACCGCGGTCTATTTCTGTGCAAGAAACTAC3' and 5'ATAGACCGCGGTGTCCTCGTTTTTCACGCTGCTGAGTCT3'. The humanised heavy chain in EU framework 16HuEUVH YTR, was constructed by mutagenesis of framework 1, 2 and 3 of 16 HuVH in NEWM framework. The primers used for framework 1 were 5'TCTGGCTACACCTTCACCACTGCTGGAATG3' and 5'GAAGGTGTAGCCAGACGCC TTGCAGCTCACTTTCACGCTGCTGCCAGGTTTCTTCACCTCTGCACCGCTCTGCTGC AGTTGGAC3'. The primers used for framework 2 were 5'AGGTCTTGAGTGGATGGGATGGATAAAC3' and 5'ATCCACTCAAGACCTTGTCCAGGTGCCTGTCTCACCCA3'. The mutagenesis of framework 3 was done in two stages. The primers used were 5'GACACCGCGTTCTATTTTTGTGCAAGAAACTAC3' and 5'ATAGAACGCGGTGTCCTCGGATCTCAGGCTGCTGAGTTC CATGTACAACTGGTTCTTGCTG3', and 5'GTACATGGAACTCAGCAGCCT3' and 5'CTGAGTTCCATGTACGCCGTGTTCGTGCTCTCGTCTGCCGTAATTGTCACTCTTC CC3'. The nucleotide sequences of the reshaped light chain and the different reshaped heavy chains are shown in figure 6.

The modified variable regions were cloned into the expression vector pSVgpt as before. Individual heavy chain plasmids were cotransfected with the original reshaped light chain plasmid into NSO cells. Antibody producing cell clones were selected and expanded. The reshaped antibodies purified were Reshaped HCMV16 ATAF, Reshaped HCMV16 ATAFSTAY, Reshaped HCMV16 ATAFFAFSL, Reshaped HCMV16 ATAFKNE and Reshaped HCMV16 YTR.

### EXAMPLE 2-HCMV SPECIFIC NEUTRALISATION BY RESHAPED ANTIBODIES

The reshaped HCMV16 antibodies, the chimeric HCMV16 antibody and the hybrid HCMV16 MuVH/HuVK antibody were tested in a neutralisation assay against HCMV strain AD169 (American Type Culture Collection, Rockwell, Maryland, USA) cultured in human embryo lung fibroblasts. The cells were grown in 48 well plates (Costar) to form confluent monolayers. The antibody preparations were serially diluted two fold to give concentrations from 100µg/ml to 0.8µg/ml in Minimal Essential Medium (MEM) supplemented with 2% heat inactivated fetal calf serum (HFCS), HEPES and antibiotics (MEM/2/D) (Life Technologies Ltd., Paisley, U.K.). Virus diluted in MEM/2/D, equivalent to 50 plaque forming units (pfu) per well, was added to the diluted antibody preparations to give final antibody concentrations of 50pg/ml to 0.4µg/ml. After incubation for 60 min at 37°C in a 5% CO₂ atmosphere, 100µl of the antibody/virus mixtures was added to each well of confluent fibroblasts in triplicate for each dilution. The plates were incubated for 60 min at 37°C in 5% CO₂ with occasional shaking. Each well was then washed once with MEM/2/D and overlayed with lml of 1% methylcellulose in MEM supplemented with 30% Lebovitz's L-15 medium, 5% HFCS, sodium bicarbonate, L-glutamine and antibiotics (Life Technologies Ltd., Paisley, U.K). The plates were incubated for 14 days at 37°C in 5% CO₂. To fix the cells, lml of 75% methanol/25% acetic acid was added to each well and the plates left at room temperature for 30 min, then washed three times with tap water. 1ml of staining solution, 0.5% crystal violet, 50% ethanol, 13.5% formalin in PBS, was added to each well and the plates left at room temperature for 30 min, after which they were washed with tap water until no dye remained.

The numbers of plaques in each well were counted and the mean number of plaque forming units (pfu) per well calculated for each dilution of the antibody preparations. Samples from a human seronegative serum pool and a human seropositive serum pool were included as controls. The control human seronegative serum pool did not neutralise virus infectivity at any dilution, while the control human seropositive serum pool neutralised infectivity well, with significant plaque reduction down to a 1/80 to 1/160 dilution. The neutralising titer was defined as the antibody concentration at which 50% plaque reduction was obtained and was determined graphically. The results from two separate experiments are shown in Tables 1 and 2.

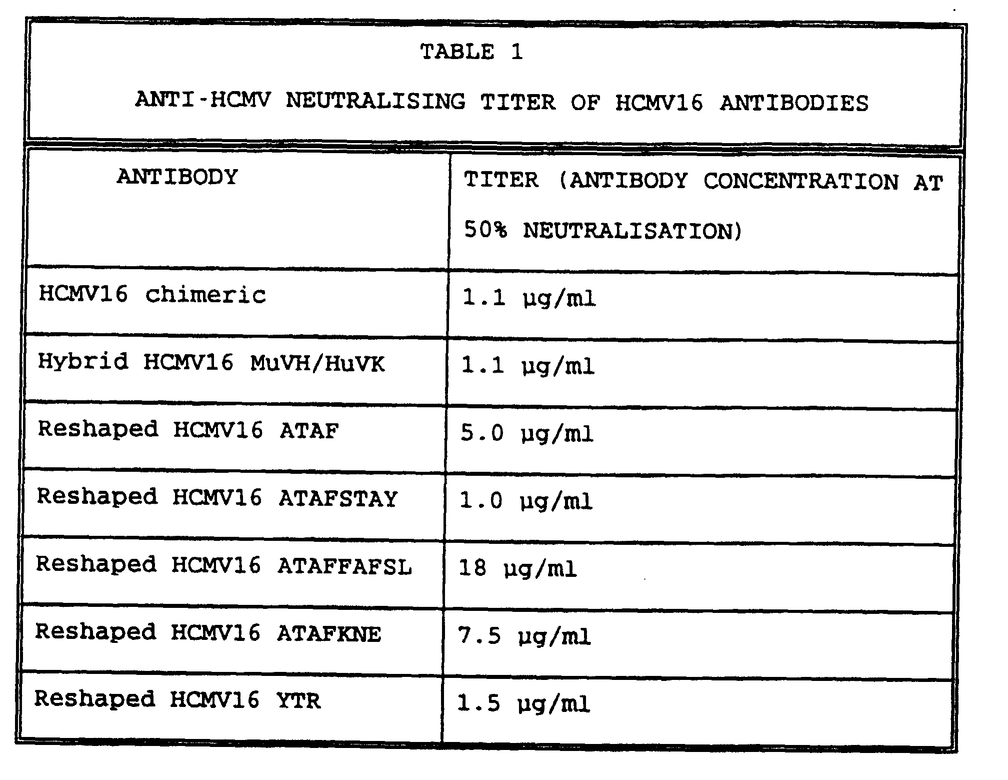

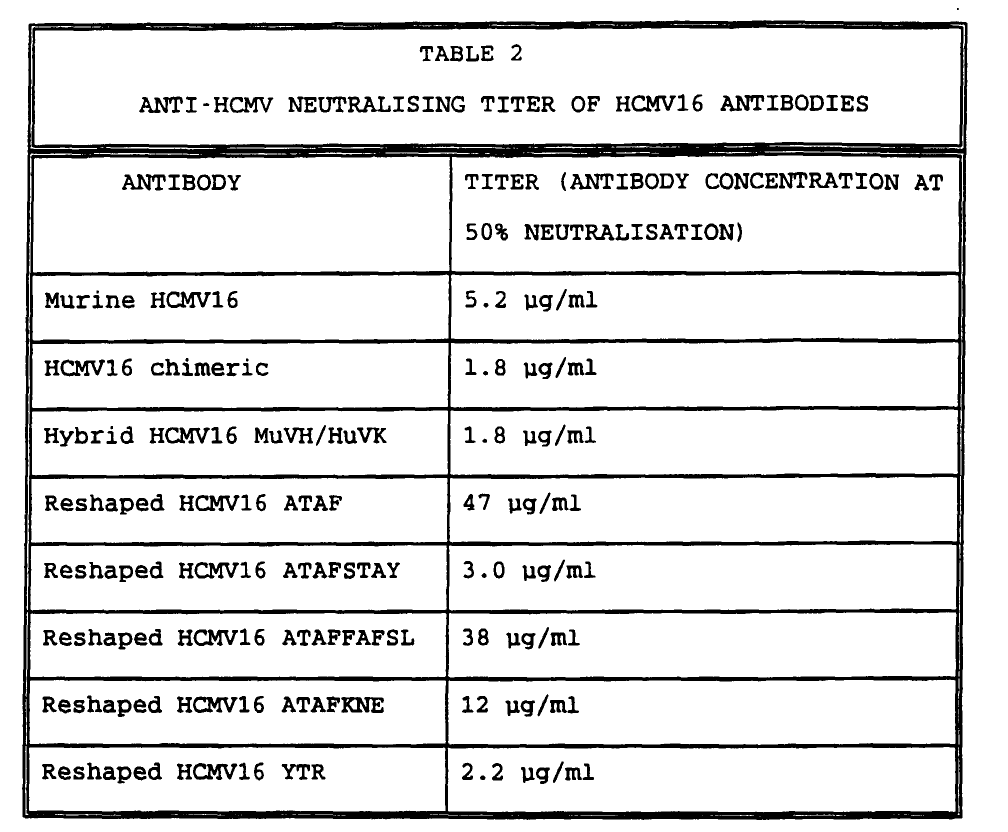

Two of the reshaped antibodies, Reshaped HCMV16 ATAFSTAY and Reshaped HCMV16 YTR neutralised HCMV virus with similar efficiency to the control chimeric antibody and these were further studied for specificity.

### EXAMPLE 3-SPECIFICITY OF ALTERED ANTIBODIES AGAINST HCMV

The two reshaped antibodies, Reshaped HCMV16 ATAFSTAY and Reshaped HCMV16 YTR were tested in an immunofluorescence assay for binding to clinical strains of HCMV.

Clinical isolates from 5 renal transplant recipients and 8 AIDS patients were propagated in human embryo lung fibroblast cells for three to five passages. When a cytopathic effect was observed the supernatant fluid containing the HCMV was harvested and spun down at 500g for 30 min to remove cellular debris. The clarified virus was added to fresh monolayers of fibroblast cells grown in 8 well chamber slides (Labtek) and incubated at 37°C for 60 min to allow the virus to adsorb. The inoculum was removed and replaced with fresh medium and the cells incubated at 37°C in a 5% CO₂ atmosphere until a cytopathic effect was observed. The cells were then washed 3 times in PBS and allowed to dry in air before fixing in acetone for 5 min at -20°C. The slides were allowed to dry in air then wrapped in cling film and stored at -20°C.

The antibodies Reshaped HCMV16 ATAFSTAY (192µg/ml) and Reshaped HCMV16 YTR (240µg/ml) were titrated in immunofluorescence against the laboratory strain of HCMV, AD169. Optimal dilutions were found to be 1/100 and 1/25 respectively. These dilutions were employed for the analysis of clinical strains.

The slides were thawed and washed with PBS. Nonspecific binding sites were blocked by the addition of a 1/5 dilution of normal rabbit serum for 60 min at 37°C. The slides were washed twice with PBS for 5 min before incubation with the appropriate dilution of reshaped antibody for 60min at 37°C. After washing in PBS as before, a 1/40 dilution of Fab₂ goat anti-human IgG gamma chain, FITC conjugate (Sigma Chemical Co. Ltd., Poole, U.K.) was added to the slides for 60 min at 37°C. Slides were washed twice with PBS then dryed in air. After mounting with Citifluor the slides were examined with an Olympus epifluorescence microscope, photographed using T-max film and the fluorescence scored. The results are shown in Table 3.

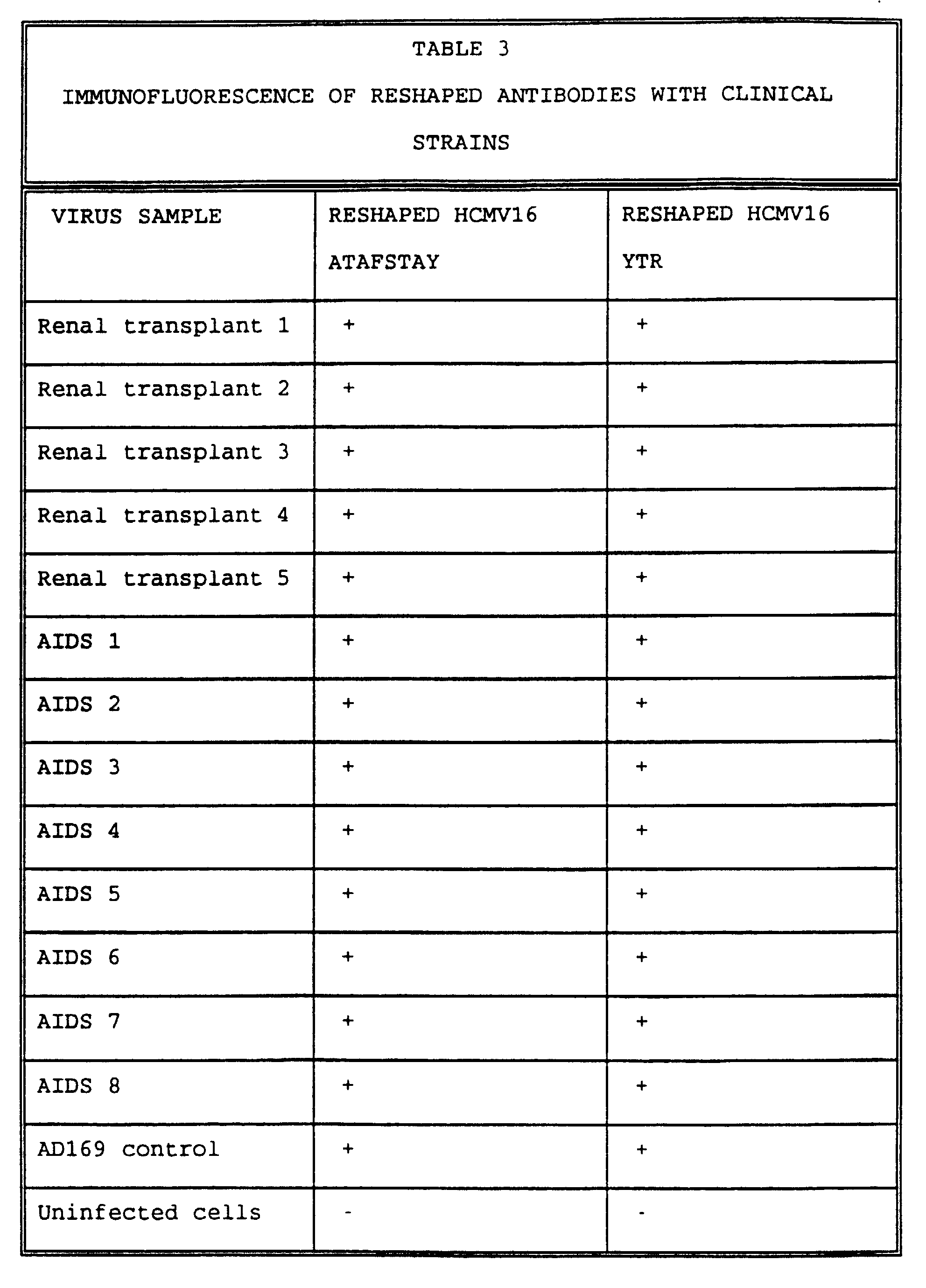

Both the Reshaped HCMV16 ATAFSTAY and the Reshaped HCMV16 YTR antibodies were shown to bind to the HCMV isolates from AIDS patients and transplant recipients. Therefore, the neutralising epitope on HCMV glycoprotein gH recognised by these altered antibodies is conserved between different clinical strains of HCMV. Therefore, these altered antibodies provide a generally applicable reagent for therapy and prophylaxis of HCMV in a clinical situation.

### EXAMPLE 4-NEUTRALISATING ACTIVITY OF ALTERED ANTIBODIES AGAINST CLINICAL STRAINS OF HOW

The two reshaped antibodies were tested for neutralisation of clinical isolates from 4 AIDS patients and 5 renal transplant patients, with the laboratory strains AD169 and Towne as controls. The ED₅₀ were calculated by the Reed-Muench method (Thorpe et al, 1987) and are shown in Table 4.

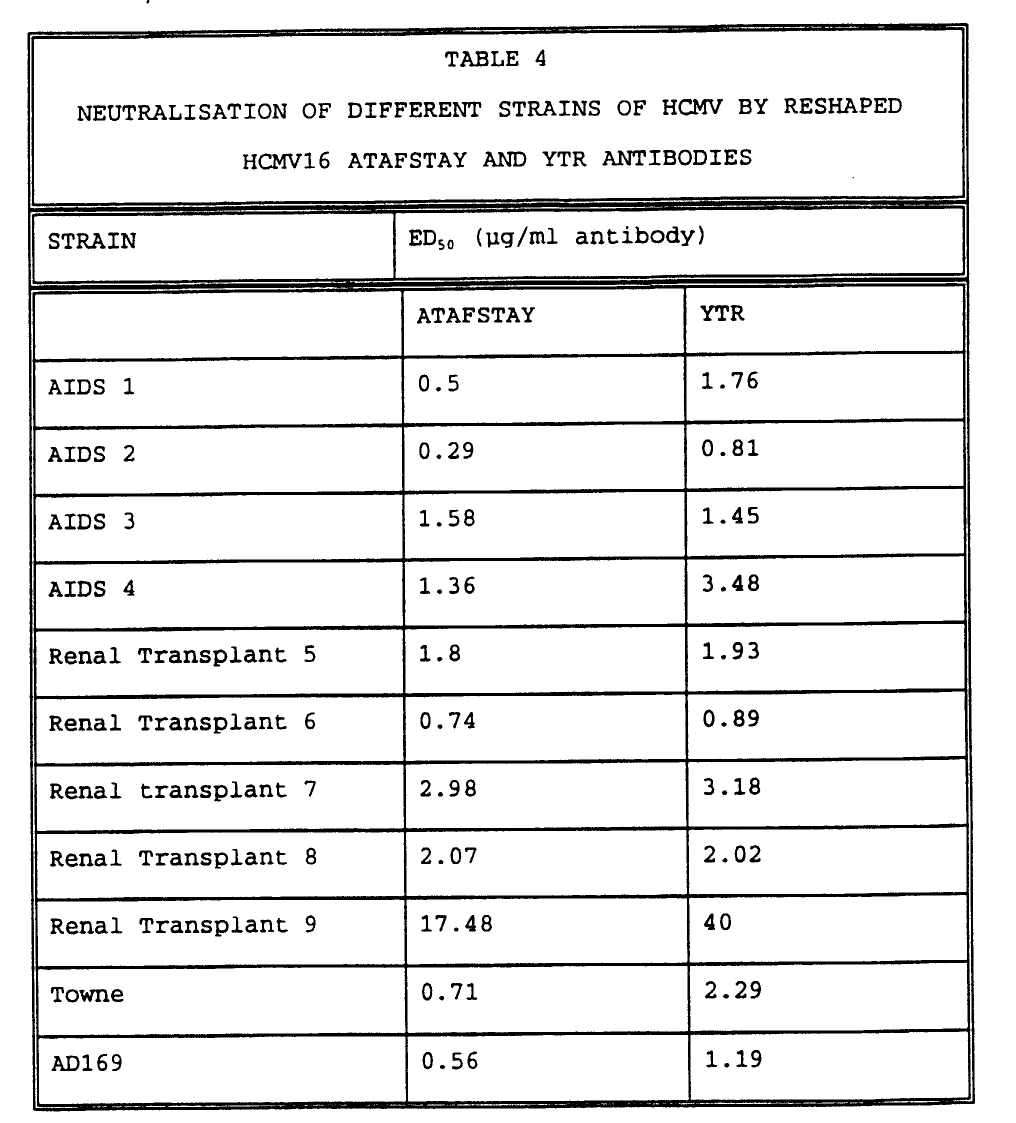

The reshaped antibodies neutralised the isolates from AIDS and renal transplant patients at equivalent concentrations to the laboratory strains, AD169 and Towne, apart from one renal transplant strain which required 40 times as much antibody. Therefore, the neutralising epitope on HCMV glycoprotein gH recognised by the reshaped antibodies is conserved between a variety of different clinical strains of HCMV.

All the references mentioned above are hereby incorporated by reference.

## Claims

1. A method of making a monoclonal antibody, or antigen-binding fragment thereof, having the HCMV-neutralising specificity of antibody HCMV16, which comprises grafting complementarity determining regions (CDRs) derived from a non-human antibody having said specificity into framework region (FRs) derived from one or more human antibodies, wherein the antibody or antigen-binding fragment has a heavy chain variable region amino acid sequence of 16HuVH ATAFSTAY or 16HuVH EUYTR as set out in figure 5.

2. A method according to claim 1 wherein the CDRs are derived from the sequences as set out in figure 1 and/or figure 2.

3. A method according to claim 1 or claim 2 wherein the heavy chain FR sequences are derived from NEWM and/or EU, and/or the light chain FR sequences are derived from REI.

4. A method according to any one of claims 1 to 3 in which the heavy chain FRs 1-3 are derived from EU and FR4 is derived from NEWM.

5. A method according to any one of the preceding claims wherein the light chain CDRs and FRs are as depicted in figure 5 for 16HuVK.

6. A monoclonal antibody or antigen-binding fragment thereof, having the HCMV neutralising specificity of antibody HCMV16, and which has CDRs derived from a non-human antibody and FRs derived from one or more human antibodies, the FRs of the heavy chain being derived from the human antibodies NEWM and/or EU, wherein the antibody or antigen-binding fragment has a heavy chain variable region amino acid sequence of 16HuVH ATAFSTAY or 16HuVH EUYTR as set out in figure 5.

7. A monoclonal antibody or antigen-binding fragment thereof, according to claim 6 having CDRs derived from the sequences as set out in figure 1 and/or figure 2.

8. A monoclonal antibody or antigen-binding fragment thereof according to claim 6 or claim 7 in which the heavy chain FRs 1-3 are derived from EU and FR4 is derived from NEWM.

9. A monoclonal antibody or antigen-binding fragment thereof according to any one of claims 6 to 8 wherein the light chain FR sequences are derived from the human antibody REI.

10. A monoclonal antibody or antigen-binding fragment thereof of any one of claims 6 to 8 or obtainable by the method of any one of claims 1 to 5 for pharmaceutical use.

11. Use of a monoclonal antibody or antigen-binding fragment thereof of any one of claim 6 to 8 or obtainable by the method of any one of claims 1 to 5 for the preparation of a medicament for the therapy or prophylaxis of HCMV.

12. Use of a monoclonal antibody or antigen-binding fragment thereof of any one of claims 6 to 8, or obtainable by the method of any one of claims 1 to 5 for the preparation of a medicament for the neutralisation of HCMV.

13. Use of a monoclonal antibody or antigen-binding fragment thereof of any one of claims 6 to 8, or obtainable by the method of any one of claims 1 to 5 for the design and synthesis of peptide or non-peptide compounds which are functional mimetics of the antibody.

14. Use of a monoclonal antibody or antigen-binding fragment thereof of any one of claims 6 to 8, or obtainable by the method of any one of claims 1 to 5 in the preparation of a medicament wherein the antibody is in conjunction with another antibody or drug.

15. A monoclonal antibody or antigen-binding fragment thereof of any one of claims 6 to 8, or obtainable by the method of any one of claims 1 to 5, wherein the antibody is attached to an effector or reporter molecule.

16. A process for producing a monoclonal antibody, or antigen-binding fragment thereof, having the HCMV-neutralising specificity of antibody HCMV16, which method comprises culturing a cell containing recombinant DNA expressing said antibody, wherein the DNA encodes an antibody of any one of claims 6 to 8 or has been prepared as part of the method of any one of claims 1 to 5.

## Patentansprüche

1. Verfahren zur Herstellung eines monoklonalen Antikörpers oder Antigen-bindenden Fragments davon mit der HCMV-neutralisierenden Spezifität des Antikörpers HCMV16, das die Verpflanzung von komplementaritätsbestimmenden Regionen (CDRs), die von einem nicht-menschlichen Antikörper mit dieser Spezifität stammen, in die von einem oder mehreren menschlichen Antikörpern stammende Gerüstregion (FRs) umfaßt, wobei der Antikörper oder das Antigen-bindende Fragment die Aminosäuresequenz der variablen Region einer schweren Kette von 16HuVH ATAFSTAY oder 16HuVH EUYTR, wie in Fig. 5 gezeigt, aufweist.

2. Verfahren nach Anspruch 1, wobei die CDRs von den in Fig. 1 und/oder Fig. 2 gezeigten Sequenzen stammen.

3. Verfahren nach Anspruch 1 oder 2, wobei die Sequenzen der Gerüstregion der schweren Kette von NEWM und/oder EU stammen und/oder die Sequenzen der Gerüstregion der leichten Kette von REI.

4. Verfahren nach einem der Ansprüche 1 bis 3, wobei FR1, FR2 und FR3 der schweren Kette von EU stammen und FR4 von NEWM stammt.

5. Verfahren nach einem der vorstehenden Ansprüche, wobei die CDRs und FRs der leichten Kette wie in Fig. 5 für 16HuVK dargestellt sind.

6. Monoklonaler Antikörper oder Antigen-bindendes Fragment davon mit der HCMV-neutralisierenden Spezifität des Antikörpers HCMV16, mit von einem nicht-menschlichen Antikörper stammenden CDRs und von einem oder mehreren menschlichen Antikörpern stammenden FRs, wobei die FRs der schweren Kette von den menschlichen Antikörpern NEWM und/oder EU stammen und wobei der Antikörper oder das Antigen-bindende Fragment die Aminosäuresequenz der variablen Region einer schweren Kette von 16HuVH ATASTAY oder 16HuVH EUYTR, wie in Fig. 5 gezeigt, aufweist.

7. Monoklonaler Antikörper oder Antigen-bindendes Fragment davon nach Anspruch 6 mit CDRs, die von den in Fig. 1 und/oder Fig. 2 gezeigten Sequenzen stammen.

8. Monoklonaler Antikörper oder Antigen-bindendes Fragment davon nach Anspruch 6 oder 7, wobei die FR1, FR2 und FR3 der schweren Kette von EU stammen und FR4 von NEWM stammt.

9. Monoklonaler Antikörper oder Antigen-bindendes Fragment davon nach einem der Ansprüche 6 bis 8, wobei die FR-Sequenzen der leichten Kette von dem menschlichen Antikörper REI stammen.

10. Monoklonaler Antikörper oder Antigen-bindendes Fragment davon nach einem der Ansprüche 6 bis 8 oder erhältlich nach dem Verfahren nach einem der Ansprüche 1 bis 5 für eine pharmazeutische Verwendung.

11. Verwendung eines monoklonalen Antikörpers oder Antigen-bindenden Fragments davon nach einem der Ansprüche 6 bis 8 oder erhältlich nach dem Verfahren nach einem der Ansprüche 1 bis 5 zur Herstellung eines Medikaments zur Therapie oder Prophylaxe von HCMV.

12. Verwendung eines monoklonalen Antikörpers oder Antigen-bindenden Fragments davon nach einem der Ansprüche 6 bis 8 oder erhältlich nach dem Verfahren nach einem der Ansprüche 1 bis 5 zur Herstellung eines Medikaments zur Neutralisierung von HCMV.

13. Verwendung eines monoklonalen Antikörpers oder Antigen-bindenden Fragments davon nach einem der Ansprüche 6 bis 8 oder erhältlich nach dem Verfahren nach einem der Ansprüche 1 bis 5 für den Entwurf und die Synthese von Peptidverbindungen oder Nicht-Peptidverbindungen, die funktionelle Nachahmer des Antikörpers sind.

14. Verwendung eines monoklonalen Antikörpers oder Antigen-bindenden Fragments davon nach einem der Ansprüche 6 bis 8 oder erhältlich nach dem Verfahren nach einem der Ansprüche 1 bis 5 zur Herstellung eines Medikaments, wobei der Antikörper in Verbindung mit einem weiteren Antikörper oder Arzneimittel vorliegt.

15. Monoklonaler Antikörper oder Antigen-bindendes Fragment davon nach einem der Ansprüche 6 bis 8 oder erhältlich nach dem Verfahren nach einem der Ansprüche 1 bis 5, wobei der Antikörper an einen Effektor oder ein Reportermolekül angeknüpft ist.

16. Verfahren zur Herstellung eines monoklonalen Antikörpers oder Antigen-bindenden Fragments davon mit der HCMV-neutralisierenden Spezifität des Antikörpers HCMV16, wobei das Verfahren das Züchten einer Zelle umfaßt, die den Antikörper exprimierende rekombinante DNA enthält, wobei die DNA einen Antikörper nach einem der Ansprüche 6 bis 8 kodiert oder als Teil des Verfahrens nach einem der Ansprüche 1 bis 5 hergestellt wurde.

## Revendications

1. Méthode de production d'un anticorps monoclonal, ou son fragment liant un antigène, ayant la spécificité de neutraliser HCMV de l'anticorps HCMV16, qui comprend la greffe de régions déterminant la complémentarité (CDR) dérivées d'un anticorps non-humain ayant ladite spécificité dans la région de charpente (FR) dérivée d'un ou plusieurs anticorps humains où l'anticorps ou le fragment liant l'antigène a une séquence d'acides aminés de sa région variable de sa chaîne lourde de 16HuVH ATAFSTAY ou 16HuVH EUYTR comme indiquée à la figure 5.

2. Méthode selon la revendication 1 où les CDR sont dérivées des séquences telles qu'indiquées à la figure 1 et/ou à la figure 2.

3. Méthode selon la revendication 1 ou la revendication 2 où les séquences de FR de la chaîne lourde sont dérivées de NEWM et/ou EU, et/ou les séquences de FR de la chaîne légère sont dérivées de REI.

4. Méthode selon l'une quelconque des revendications 1 à 3 où les FR 1-3 de la chaîne lourde sont dérivées de EU et FR4 est dérivée de NEWM.

5. Méthode selon l'une quelconque des revendications précédentes où les CDR et les FR de la chaîne légère sont telles que représentées à la figure 5 pour 16HuVK.

6. Anticorps monoclonal ou son fragment liant un antigène ayant la spécificité de neutralisation de HCMV de l'anticorps HCMV16, et qui a des CDR dérivées d'un anticorps non-humain et des FR dérivées d'un ou plusieurs anticorps humains, les FR de la chaîne lourde étant dérivées des anticorps humains NEWM et/ou EU, où l'anticorps ou le fragment liant l'antigène a une séquence d'acides aminés de la région variable de la chaîne lourde de 16HuVH ATAFSTAY ou 16HuVH EUYTR tel qu'indiquée à la figure 5.

7. Anticorps monoclonal ou son fragment liant un antigène selon la revendication 6 ayant des CDR dérivées de séquences telles qu'indiquées à la figure 1 et/ou la figure 2.

8. Anticorps monoclonal ou son fragment liant un antigène selon la revendication 6 ou la revendication 7 dans lequel les FR 1-3 de la chaîne lourde sont dérivées de EU et FR4 est dérivée de NEWM.

9. Anticorps monoclonal ou son fragment liant un antigène selon l'une quelconque des revendications 6 à 8 où les séquences de FR de la chaîne légère sont dérivées de l'anticorps humain REI.

10. Anticorps monoclonal ou son fragment liant un antigène selon l'une quelconque des revendications 6 à 8 ou pouvant être obtenu par la méthode selon l'une quelconque des revendications 1 à 5 pour un usage pharmaceutique.

11. Utilisation d'un anticorps monoclonal ou de son fragment liant un antigène selon l'une quelconque des revendications 6 à 8 ou pouvant être obtenu par la méthode selon l'une quelconque des revendications 1 à 5 pour la préparation d'un médicament pour la thérapie ou la prophylaxie de HCMV.

12. Utilisation d'un anticorps monoclonal de son fragment liant un antigène selon l'une quelconque des revendications 6 à 8 ou pouvant être obtenu par la méthode selon l'une quelconque des revendications 1 à 5 pour la préparation d'un médicament pour la neutralisation de HCMV.

13. Utilisation d'un anticorps monoclonal ou de son fragment liant un antigène selon l'une quelconque des revendications 6 à 8 ou pouvant être obtenu par la méthode selon l'une quelconque des revendications 1 à 5 pour la conception et la synthèse de composés peptidiques ou non peptidiques qui sont des mimétiques fonctionnels de l'anticorps.

14. Utilisation d'un anticorps monoclonal ou de son fragment liant un antigène selon l'une quelconque des revendications 6 à 8 ou bien pouvant être obtenu par la méthode selon l'une quelconque des revendications 1 à 5, dans la préparation d'un médicament où l'anticorps est en conjonction avec un autre anticorps ou une drogue.

15. Anticorps monoclonal ou son fragment liant un antigène selon l'une quelconque des revendications 6 à 8 ou bien pouvant être obtenu par la méthode selon l'une quelconque des revendications 1 à 5 où l'anticorps est attaché à une molécule effecteur ou reporteur.

16. Procédé de production d'un anticorps monoclonal, ou son fragment liant un antigène, ayant la spécificité de neutralisation de HCMV de l'anticorps HCMV16, laquelle méthode comprend la culture d'une cellule contenant un ADN recombinant exprimant ledit anticorps, où ledit ADN code pour un anticorps selon l'une quelconque des revendications 6 et 8 ou a été préparé comme partie de la méthode selon l'une quelconque des revendications 1 à 5.
